(19) Europäisches Patentamt
European Patent Office
Office européen des brevets

(11) Veröffentlichungsnummer: **0 129 153**
**B1**

(12) # EUROPÄISCHE PATENTSCHRIFT

(45) Veröffentlichungstag der Patentschrift: **20.11.86**

(51) Int. Cl.[4]: **C 07 D 233/50**, C 07 D 405/12, A 61 K 31/415, A 61 K 31/33

(21) Anmeldenummer: **84106487.6**

(22) Anmeldetag: **06.06.84**

(54) **Neue 1-Acetonyl-2-(phenylimino)-imidazolidine, deren Säureadditionssalze, diese enthaltende Arzneimittel und Verfahren zu deren Herstellung.**

(30) Priorität: **13.06.83 DE 3321282**

(43) Veröffentlichungstag der Anmeldung: **27.12.84 Patentblatt 84/52**

(45) Bekanntmachung des Hinweises auf die Patenterteilung: **20.11.86 Patentblatt 86/47**

(84) Benannte Vertragsstaaten: **AT BE CH DE FR IT LI LU NL SE**

(56) *Entgegenhaltungen:*
**DE - A - 2 523 103**

(73) Patentinhaber: **BOEHRINGER INGELHEIM KG, D-6507 Ingelheim am Rhein (DE)**

(72) Erfinder: **Esser, Franz, Dr., Albrecht-Dürer-Strasse 24, D-6507 Ingelheim/Rhein (DE)**
Erfinder: **Köppe, Herbert, Dr., Neuweg 72, D-6507 Ingelheim/Rhein (DE)**
Erfinder: **Abele, Wolfgang, Dr., Kirchstrasse 2 a, D-6531 Waldalgesheim (DE)**
Erfinder: **Stockhaus, Klaus, Dr., Pfarrer-Heberer-Strasse 35, D-6530 Bingen/Rhein (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 129 153 B1

ACTORUM AG

**Beschreibung**

Die Erfindung betrifft neue 1-Acetonyl-2-(phenylimino)-imidazolidin-derivate der allgemeinen Formel I

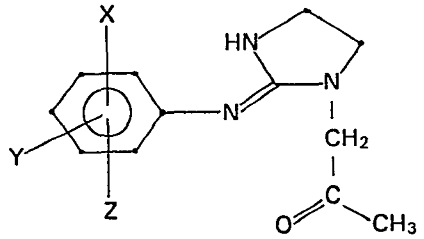

I

sowie deren physiologisch verträgliche Säureadditionssalze mit wertvollen therapeutischen Eigenschaften.

In der Formel I bedeutet X, Y und Z, die gleich oder verschieden sein können, ein Wasserstoff- oder Halogenatom, eine Alkyl-, Halogenalkyl-, Alkoxi-, Halogenalkoxid-, Alkylthio- oder Halogenalkylthiogruppe oder Y und Z zusammen einen Alkylendioxirest. Bevorzugt bezeichnet X ein Fluor-, Chlor- oder Bromatom oder eine Methoxi-, Methylthio-, Trifluormethoxi-, Pentafluorethoxi- oder Methylgruppe, Y ein Wasserstoff-, Fluor-, Chlor-, Bromatom oder eine Methyl- oder Trifluormethylgruppe und Z ein Wasserstoffatom oder in Verbindung mit Y eine Ethylendioxigruppe.

Die Herstellung von Verbindungen der allgemeinen Formel I kann nach verschiedenen Verfahren erfolgen, wobei sich das folgende besonders bewährt hat:

Das substituierte 1-Propargyl-2-(phenylimino)-imidazolidin der allgemeinen Formel II

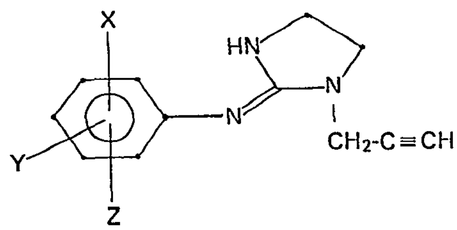

II

worin X, Y und Z die oben angegebenen Bedeutungen besitzen, wird mit oder ohne Anwendung von Schwermetallkatalyse zur entsprechenden Acetonylverbindung der allgemeinen Formel I hydratisiert.

Die Umsetzung wird zweckmässigerweise in wässrigschwefelsaurem Milieu bei Temperaturen zwischen 20° und 100°, vorzugsweise bei 60°C durchgeführt, wobei als Schwermetallkatalysatoren bevorzugt Quecksilber-II-Salze eingesetzt werden. Die Isolierung der Substanz erfolgt nach Alkalisieren der Reaktionslösung mit 5 n Natronlauge durch Extraktion mit einem organischen Lösungsmittel, vorzugsweise mit Essigester und anschliessendem Abdampfen des Lösungsmittels.

Ausgangsverbindungen der allgemeinen Formel II sind in der DE-A 2 523 103 beschrieben. Man erhält sie durch Umsetzung von Metallsalzen der 2-Phenylimino-imidazolidine mit Propargylhalogeniden.

Die erfindungsgemässen 1-Acetonyl-2-(phenylimino)-imidazolidin-derivate der allgemeinen Formel I können auf übliche Weise in ihre physiologisch verträglichen Säureadditionssalze überführt werden. Zur Salzbildung geeignete Säuren sind beispielsweise Mineralsäuren wie Salzsäure, Bromwasserstoffsäure, Schwefelsäure, Phosphorsäure, Salpetersäure, oder organische Säuren wie Essigsäure, Propionsäure, Buttersäure, Capronsäure, Valeriansäure, Oxalsäure, Malonsäure, Weinsäure, Zitronensäure, Äpfelsäure, Benzoesäure, Zimtsäure, Ascorbinsäure, Methansulfonsäure, Ethanphosphonsäure, 8-Chlortheophyllin und dergleichen.

Die neuen Verbindungen der allgemeinen Formel I sowie deren Säureadditionssalze haben wertvolle pharmakologische, insbesondere analgetische Eigenschaften. Sie können daher bei der Behandlung der verschiedenen Erscheinungsformen von Schmerzzuständen Anwendung finden. Die Verbindungen der allgemeinen Formel I und deren Säureadditionssalze können enteral oder auch parenteral angewandt werden. Die Dosierung liegt bei 0,1 bis 80 mg, vorzugsweise 1 bis 30 mg.

Die Verbindungen der allgemeinen Formel I oder ihrer Säureadditionssalze können auch mit andersartigen Wirkstoffen zum Einsatz gelangen. Geeignete galenische Darreichungsformen sind beispielsweise Tabletten, Kapseln, Zäpfchen, Lösungen, Pulver oder Ampullen. Hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die analgetische Wirkung der erfindungsgemässen Verbindungen der allgemeinen Formel I wurde im Writhing-Test an der Maus nach subcutaner Application festgestellt. Beispielsweise kann nach s.c.-Applikation von 0,3 mg pro kg 1-Acetonyl-2-(2,4-dichlorphenylimino)-imidazolidin-hydrobromid der Schmerzeffekt zu 50% gehemmt werden.

Die folgenden Beispiele erläutern die Erfindung ohne sie zu beschränken.

*A. Herstellungsbeispiele*

*Beispiel 1*

*1-Acetonyl-2-(2,4-dichlorphenylimino)-imidazolidin-hydrobromid*

Zu 21,5 g 1-Propargyl-2-(2,4-dichlorphenylimino)-imidazolidin-oxalat (0,06 Mol) werden 96 ml $H_2O$, 3,84 ml konz. $H_2SO_4$ und 0,6 g $HgSO_4$ gegeben und 7 Stunden bei 60°C gerührt. Dünnschichtchromatogramm-Kontrolle zeigt vollständige Umsetzung an. Vom $HgSO_4$ wird abfiltriert, das klare Filtrat mit Wasser auf 400 ml verdünnt, mit 5 n NaOH alkalisch gestellt und 2 × mit Essigester extrahiert. Die Wasserphase wird verworfen. Nach Filtration wird die organische Phase bis zur Gewichtskonstanz getrocknet, wobei 16,8 g Öl, entsprechend 97,8% Rohausbeute erhalten werden. Die 16,8 g der erhaltenen Base werden mit alkoholischer HBr in Lösung gebracht, filtriert und im Filtrat durch Etherzugabe als Hydrobromid zur Kristallisation gebracht, abgesaugt, mit Ether gewaschen und getrocknet, wobei 17,0 g 1-Acetonyl-2-(2,4-dichlor-phenylimino)-imidazolidin-hydrobromid als Reinprodukt, entsprechend 77,3% der Theorie isoliert werden.

Fp.: 195 - 196°C.

*Beispiel 2*

*1-Acetonyl-2-(3-fluor-4-methylphenylimino)-imidazolidin Toluolsulfonat*

Durch analoges Vorgehen wie in Beispiel 1 werden aus 6,94 g 1-Propargyl-2-(3-fluor-4-methylphenylimino)-imidazolidin 5,5 g der entsprechenden Acetonylverbindung als Toluolsulfonat erhalten.

Ausbeute: 43,5% der Theorie

Fp.: 136 - 137°C

*Beispiel 3*

*1-Acetonyl-2-(2-brom-5-chlorphenylimino)-imidazolidin*

Analog zu Beispiel 1 werden aus 6,25 g 1-Propargyl-2-(2-brom-5-chlorphenylimino)-imidazolidin 2,0 g der entsprechenden Acetonylverbindung als freie Base erhalten.

Ausbeute: 30% der Theorie

Fp.: — (Öl)

Dünnschichtchromatogramm: Fliessmittel Toluol : Dioxan : EtOH: konz. $NH_3$ = 50:40:15:5

$R_f$-Wert 0,65.

*Beispiel 4*

*1-Acetonyl-2-(4-chlorphenylimino)-imidazolidin Hydrobromid*

Analog zu Beispiel 1 werden ausgehend von 6 g 1-Propargyl-2-(4-chlorphenylimino)-imidazolidin 5,7 g der entsprechenden Acetonylverbindung als Hydrobromid isoliert.

Ausbeute: 66% der Theorie

Fp.: 149 - 151°C

*Beispiel 5*

*1-Acetonyl-2-(4-chlor-2-methoxiphenylimino)-imidazolidin*

Analog zu Beispiel 1 werden aus 5 g 1-Propargyl-2-(4-chlor-2-methoxiphenylimino)-imidazolidin 2,7 g der entsprechenden Acetonylverbindung als freie Base gewonnen.

Ausbeute: 50,4% der Theorie

Fp.: 83 - 85°C

*Beispiel 6*

*1-Acetonyl-2-(2-brom-4,5-ethylendioxiphenylimino)-imidazolidin*

Analog zu Beispiel 1 werden 5 g 1-Propargyl-2-(2-brom-4,5- ethylendioxiphenylimino)-imidazolidin zu 4 g der entsprechenden Acetonylverbindung als freier Base umgesetzt.

Ausbeute: 66,4% der Theorie

Fp.: 98 - 100°C

*Beispiel 7*

*1-Acetonyl-2-(2-methyl-thio-phenylimino)-imidazolidin*

Analog zu Beispiel 1 werden aus 6 g 1-Propargyl-2-(2-methyl-thio-phenylimino)-imidazolidin 2,2 g der entsprechenden Acetonylverbindung als freie Base gewonnen.

Ausbeute: 33% der Theorie

Fp.: — (Öl)

Dünnschichtchromatogramm: Fliessmittel wie in Beispiel 3,

$R_f$ = 0,63.

*Beispiel 8*

*1-Acetonyl-2-(5-chlor-2-trifluormethoxiphenylimino)-imidazolidin*

Analog zu Beispiel 1 werden 1,75 g 1-Propargyl-2-(5-chlor-2-trifluormethoxiphenylimino)-imidazolidin in 0,8 g der entsprechenden Acetonylverbindung als freie Base überführt.

Ausbeute: 43% der Theorie

Fp.: — (Öl)

Dünnschichtchromatogramm: Fliessmittel wie in Beispiel 3,

$R_f$ = 0,67.

*Beispiel 9*

*1-Acetonyl-2-(2-pentafluorethoxi-5-fluorphenylimino)-imidazolidin*

Analog zu Beispiel 1 werden aus 3,3 g 1-Propargyl-2-(2-pentafluorethoxi-5-fluorphenylimino)-imidazolidin 1,7 g der entsprechenden Acetonylverbindung als freie Base hergestellt.

Ausbeute: 49% der Theorie

Dünnschichtchromatogramm: Fliessmittel wie in Beispiel 3,

$R_f$ = 0,73.

*Beispiel 10*

*1-Acetonyl-2-(5-brom-2-methylphenylimino)-imidazolidin*

Analog zu Beispiel 1 werden aus 8,4 g 1-Propargyl-2-(5-brom-2-methylphenylimino)-imidazolidin 2,2 g der entsprechenden Acetonylverbindung in Form der freien Base gewonnen.

Ausbeute: 72% der Theorie

Fp.: 93 - 96°C

*Beispiel 11*

*1-Acetonyl-2-(2-brom-6-fluorphenylimino)-imidazolidin*

Analog zu Beispiel 1 werden aus 2,1 g 1-Propargyl-2-(2-brom-6-fluorphenylimino)-imidazolidin 1,2 g der entsprechenden Acetonylverbindung als freie Base erhalten.

Ausbeute: 60% der Theorie

Fp.: 84 - 88°C

*Beispiel 12*

*1-Acetonyl-2-(2-chlor-5-trifluormethylphenylimino)-imidazolidin*

Analog zu Beispiel 1 werden aus 5 g 1-Propargyl-2-(2-chlor-5-trifluormethylphenylimino)-imidazolidin

2,2 g der entsprechenden Acetonylverbindung als freie Base hergestellt.

Ausbeute: 40% der Theorie

Fp.: 93 - 95°C

*Beispiel 13*

*1-Acetonyl-2-(2,6-dichlorphenylimino)-imidazolidin*

Analog zu Beispiel 1 werden ausgehend von 6,2 g 1-Propargyl-2-(2,6-dichlorphenylimino)-imidazolidin 2,1 g der entsprechenden Acetonylverbindung erhalten.

Ausbeute: 32% der Theorie

Fp.: 84 - 86°C

*B. Pharmazeutische Formulierungsbeispiele*

a) *Dragées*

1 Dragéekern enthält:

| | |
|---|---|
| Wirkstoff gemäss vorliegender Erfindung | 2,0 mg |
| Milchzucker | 28,5 mg |
| Maisstärke | 17,0 mg |
| Gelatine | 2,0 mg |
| Magnesiumstearat | 0,5 mg |
| | 50,0 mg |

Herstellung:

Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpresst. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragées werden mit Bienenwachs poliert.

Dragée-Endgewicht: 100 mg.

b) *Tabletten*

| | |
|---|---|
| Wirkstoff gemäss vorliegender Erfindung | 2,0 mg |
| Milchzucker | 55,0 mg |
| Maisstärke | 38,0 mg |
| lösliche Stärke | 4,0 mg |
| Magnesiumstearat | 1,0 mg |
| | 100,0 mg |

Herstellung:

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpresst, die 2 mg Wirkstoff enthalten.

c) *Suppositorien*

1 Zäpfchen enthält:

| | |
|---|---|
| Wirkstoff gemäss vorliegender Erfindung | 1,0 mg |
| Zäpfchenmasse | 1699,0 mg |

Herstellung:

Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) *Ampullen*

| | | |
|---|---|---|
| Wirkstoff gemäss vorliegender Erfindung | | 2,0 mg |
| Natriumchlorid | | 18,0 mg |
| destilliertes Wasser | ad | 2,0 ml |

Herstellung:

Wirkstoff und Natriumchlorid werden in Wasser gelöst, die Lösung frei nach suspendierten Partikeln filtriert und in 2 ccm-Ampullen unter aseptischen Bedingungen abgefüllt. Zuletzt werden die Ampullen sterilisiert und verschlossen. Jede Ampulle enthält 2 mg Wirkstoff.

**Patentansprüche**

1. 1-Acetonyl-2-(phenylimino)-imidazolidine der allgemeinen Formel I

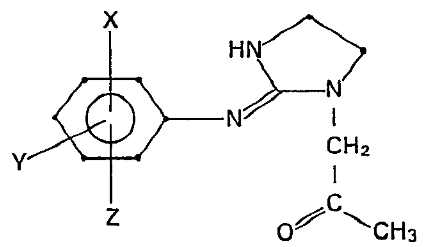

I

in der X, Y und Z, die gleich oder verschieden sein können, ein Wasserstoff- oder Halogenatom oder eine Alkyl-, Halogenalkyl-, Alkoxi-, Halogenalkoxi-, Alkylthio-, Halogenalkylthiogruppe oder Y und Z zusammen einen Alkylendioxirest bedeuten sowie deren Säureadditionssalze.

2. 1-Acetonyl-2-(phenylimino)-imidazolidine der allgemeinen Formel I nach Anspruch 1, worin X ein Fluor-, Chlor- oder Bromatom oder eine Methoxi-, Methylthio-, Trifluormethoxi-, Pentafluorethoxi- oder Methylgruppe, Y ein Wasserstoff-, Fluor-, Chlor- oder Bromatom oder eine Methyl-, oder Trifluormethylgruppe und Z ein Wasserstoffatom oder in Verbindung mit Y eine Ethylendioxigruppe bedeuten sowie deren Säureaddtionssalze.

3. Verfahren zur Herstellung von 1-Acetonyl-2-(phenylimino)-imidazolidinen der allgemeinen Formel I nach Anspruch 1 und von deren Säureadditionssalzen, dadurch gekennzeichnet, dass man ein substituiertes 1-Propargyl-2-(phenylimino)-imidazolidin der allgemeinen Formel II

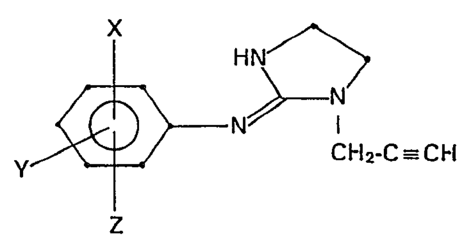

II

worin X, Y und Z die oben genannten Bedeutungen besitzen, mit oder ohne Anwendung von Schwermetallkatalyse hydratisiert und gegebenenfalls die erhaltene Verbindung in ein Säureadditionssalz überführt.

4. Verfahren nach Anspruch 3, dadurch gekennzeichnet, dass man die Umsetzung in Gegenwart von Quecksilber-II-Salzen in wässrigschwefelsaurem Milieu bei Temperaturen zwischen 20° und 100°, vorzugsweise bei 60°C durchführt.

5. Pharmazeutische Zubereitungen, dadurch gekennzeichnet, dass sie als Wirkstoffe eine oder mehrere Verbindungen der allgemeinen Formel I oder deren physiologisch verträgliche Säureadditionssalze enthalten.

6. Verfahren zur Herstellung von Arzneimitteln nach Anspruch 5, dadurch gekennzeichnet, dass man eine oder mehrere Verbindungen der allgemeinen Formel I oder deren Säureadditionssalze mit üblichen galenischen Hilfs-, Träger-, Spreng- oder Schmiermitteln oder Substanzen zur Erzielung einer Depotwirkung zu Tabletten, Kapseln, Zäpfchen, Lösungen oder Pulver formuliert.

7. Verwendung von Verbindungen der allgemeinen Formel I oder deren Säureadditionssalze nach Anspruch 1 zur Herstellung eines Arzneimittels zur Bekämpfung von Schmerzzuständen.

**Claims**

1. 1-Acetonyl-2-(phenylimino)-imidazolidines of general formula I

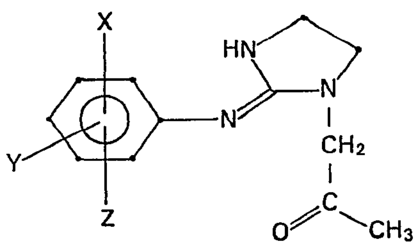

I

wherein X, Y and Z, which may be the same or different, represent, a hydrogen or halogen atom or an alkyl, haloalkyl, alkoxy, haloalkoxy, alkylthio, haloalkylthio, group or Y and Z together represent an alkylenedioxy group, and the acid addition salts thereof.

2. 1-Acetonyl-2-(phenylimino)-imidazolidines of general formula I as claimed in claim 1, wherein X represents a fluorine, chlorine or bromine atom or a methoxy, methylthio, trifluoromethoxy, pentafluoroethoxy or methyl group, Y represents a hydrogen, fluorine, chlorine or bromine atom or a methyl or trifluoromethyl group and Z represents a hydrogen atom or, in conjunction with Y, an ethylenedioxy group, and the acid addition salts thereof.

3. Process for the preparation of 1-acetonyl-2-(phenylimino)-imidazolidines of general formula I as claimed in claim 1 and of the acid addition salts thereof, characterised in that a substituted 1-propargyl-2-(phenylimino)-imidazolidine of general formula II

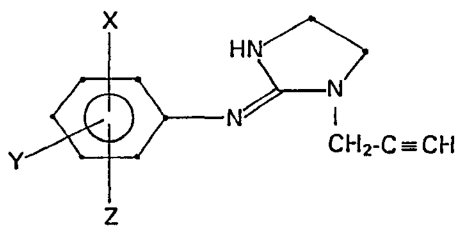

II

wherein X, Y and Z are as hereinbefore defined, is hydrated with or without the use of heavy metal catalysts and if desired the resulting compound is converted into an acid addition salt.

4. Process as claimed in claim 3. characterised in that the reaction is carried out in the presence of mercury(II)salts in an aqueous sulphuric acid medium at temperatures of between 20° and 100°, preferably at 60°C.

5. Pharmaceutical preparations, characterised in that they contain as active substance one or more compounds of general formula I or the physiologically acceptable acid addition salts thereof.

6. Process for the preparation of pharmaceutical compositions as claimed in claim 5, characterised in that one or more compounds of general formula I or the acid addition salts thereof are formulated with conventional galenic excipients, carriers, disintegrants or lubricants or substances for obtaining delayed release to form tablets, capsules, suppositories, solutions or powders.

7. Use of compounds of general formula I or of the acid addition salts thereof as claimed in claim 1 for the preparation of a pharmaceutical composition for combatting pain.

**Revendications**

1. 1-acétonyl-2-(phénylimino)-imidazolidines de formule générale I

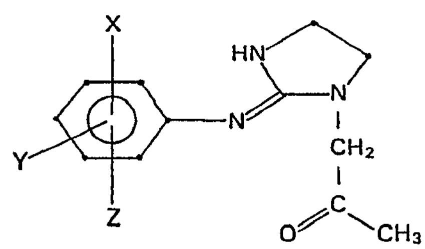

I

dans laquelle X, Y et Z qui peuvent être identiques ou différents, représentent un atome d'hydrogène ou d'halogène ou un groupe alcoyle, halogéno-alcoyle, alcoxy, halogéno-alcoxy, alcoylthio, halogéno-alcoylthio ou Y et Z représentent ensemble un radical alcoylènedioxy ainsi que leurs sels d'addition d'acides.

2. 1-acétonyl-2-(phénylimino)-imidazolidines de formule générale I selon la revendication 1, dans laquelle X représente un atome de fluor, de chlore ou de brome ou un groupe méthoxy, méthylthio, trifluorométhoxy, pentafluoroéthoxy ou méthyle, Y représente un atome d'hydrogène, de fluor, de chlore ou de brome ou un groupe méthyle ou trifluorométhyle et Z représente un atome d'hydrogène ou, en combinaison avec Y, un groupe éthylènedioxy, ainsi que leurs sels d'addition d'acides.

3. Procédé pour la préparation de 1-acétonyl-2-(phénylimino)-imidazolidines de formule générale I selon la revendication 1 et de leurs sels d'addition d'acides, caractérisé en ce qu'on hydrate une 1-propargyl-2-(phénylimino)-imidazolidine substituée de formule générale II

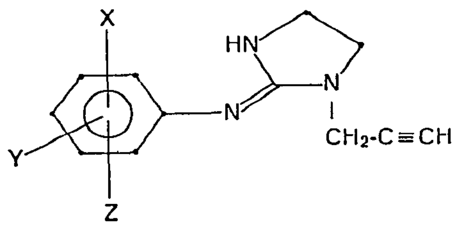

II

dans laquelle X, Y et Z possèdent les significations mentionnées plus haut, en ayant recours ou non à une catalyse par des métaux lourds et en ce qu'on transforme éventuellement le composé obtenu en un sel d'addition d'acide.

4. Procédé selon la revendication 3, caractérisé en ce qu'on effectue la réaction en présence de sels de mercure-II en milieu acide sulfurique aqueux à des températures entre 20° et 100°, de préférence à 60°C.

5. Préparations pharmaceutiques, caractérisées en ce qu'elles contiennent, en tant que substance active, un ou plusieurs composés de formule générale I ou leurs sels d'addition d'acides physiologiquement supportables.

6. Procédé pour la préparation de médicaments selon la revendication 5, caractérisé en ce que l'on formule un ou plusieurs composés de formule générale I ou leurs sels d'addition d'acides avec des adjuvants, excipients, désagrégeants ou lubrifiants ou substances pour obtenir un effet retard galéniques usuels pour donner des comprimés, capsules, suppositoires, solutions ou poudres.

7. Utilisation des composés de formule générale I ou de leurs sels d'addition d'acides selon la revendication 1 pour la préparation d'un médicament pour combattre les états douloureux.